# EUROPEAN PATENT APPLICATION

(11) **EP 3 327 009 A1**
(43) Date of publication of application: **30.05.2018**
(21) Application number: 16200228.1
(22) Date of filing: 23.11.2016
(51) Int. Cl.: C07D 251/70, C07C 275/06

(54) **METHOD FOR ALKYLATING AN AMINO COMPOUND**

(71) Applicant: Borealis Agrolinz Melamine GmbH, 4021 Linz (AT)
(72) Inventor: GABRIEL, Herbert, 4067 Marienkirchen (AT); DICKE, René, 4060 Leonding (AT)
(74) Representative: Maikowski & Ninnemann Patentanwälte Partnerschaft mbB

(57) **Abstract**

The invention relates to a method for alkylating an amino compound, characterized in that at least one triazine derivative of general formula (I) 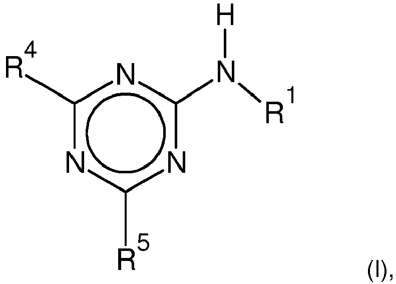 or at least one urea or urea derivative of the general formula (II) 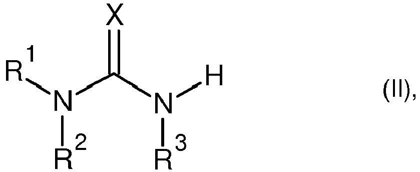 wherein
• R⁴ and R⁵ mean independently from each other Q¹ or a moiety of the formula R⁶-N-R⁷ or R⁸-N-R⁹ bound with its central nitrogen atom to the triazine ring of the structure of formula (I), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-, -OC(O)-, -C(O)- and/or -OC(O)O-,

• R¹, R², R³, R⁶, R⁷, R⁸ and R⁹ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type -C(O)O-, - OC(O)-, -C(O)- and/or -OC(O)O- and/or can be functionalized by one or multiple hydroxyl groups and/or mercapto groups, and
• X means O or S,
is reacted with at least one alcohol of the general formula (III)

R¹⁰-OH

wherein R¹⁰ means a linear or branched C₁-C₂₀-alkyl, C₅-C₂₀-cycloalkyl, or C₁-C₂₀-alkylsubstituted C₅-C₂₀-aryl, which can be in each case interrupted by one or multiple oxygen atoms, sulphur atoms, substituted nitrogen atoms and/or by one or multiple groups of the type -C(O)O-, -OC(O)-, -C(O)- and/or -OC(O)O- and/or can be functionalized by one or multiple hydroxyl groups and/or mercapto groups, wherein the reaction is carried out in the presence of at least one Ruthenate of the general formula (IV) Mₙ(RuO₄) and/or at least one Perruthenate of the general formula (V) M(RuO₄)n wherein the cation M is selected from a group comprising an alkali, earth alkali or substituted or non-substituted ammonium cation.

## Description

Several methods and processes for alkylating amino compounds such as triazines, in particular melamine, have already been described.

For example, EP 0 711 760 A1 describes an alkylation of melamine by reacting melamine in the presence of a catalyst and an atmosphere of argon, nitrogen, carbon monoxide or a mixture of hydrogen and carbon monoxide. Different metals and metal complex are mentioned as possible catalyst system such as iron, cobalt, ruthenium, palladium and other metals of the group VIII of the periodic table. A number of possible ruthenium containing complex are listed such as pentacarbonylruthenium, dodecacarbonyltriruthenium and others. Besides ruthenium chloride and ruthenium oxides are also mentioned. However, no complete conversion of the educts and no selectivity in respect to single products are achieved.

EP 1 057 821 A1 describes the alkylation of melamine with alcohols in the presence of a binary hydrogenation-dehydrogenation metal catalysts with acidic carriers and a nitrogen or hydrogen atmosphere. For example a typical catalyst used is a combination of Pd and CuO-Cr₂O₃ system. No complete conversion of the educts and no selectivity in respect to single products are achieved.

JP 2000-063360 A describes a method for alkylation of melamine in which melamine is reacted with an alcohol at high temperatures in the presence of a metal catalyst on a microporous carrier. Thereby, preferably lower alkylated products are obtained. In this method a low selectivity of the alkylated products on the one hand and the formation of cyanic acid esters as side products by hydrolysis or substitution of the amino groups on the other hand are recognized. The reaction occurs under a nitrogen, argon, hydrogen or carbon monoxide atmosphere.

The alkylation of melamine with alcohols using a Ruthenium catalyst has also been mentioned by Inage *et al* (Inage K., Yamakawa T., Shinoda S., et al., Shape selective N-alkylation of melamine using alcohol as an alkylating agent with Ru/mordenite catalyst in the liquid phase, Stud Surf Sci Catal, 130, 3465-3470, 2000.

WO 2009/121603 A1 and WO 2009/121607 A1 also refer to alkylation methods of triazines and urea compounds wherein the reaction mixture contains ammonia or a polyalcohol is used as reaction partner. A rather high selectivity in respect to the single alkylated product is obtained in both cases. However, the drawback is the rather high amount of catalyst that is required.

Thus, there is still a need for providing a method for alkylating an amino compound, in particular melamine as a triazine, that provides a high selectivity and a high yield and at the same time requires rather low catalyst amount.

The object of the invention is therefore to provide an alkylation method for amino compounds, such as triazines or urea that enables the introduction of multiple substituents in the respective amino compound in high selectivity regarding the distribution of the substituents and requires at the same time a low amount of catalyst.

This object is being solved by providing a method with the features of claim 1.

Accordingly, a method for alkylating an amino compound is provided, wherein
- at least one triazine derivative of general formula (I) 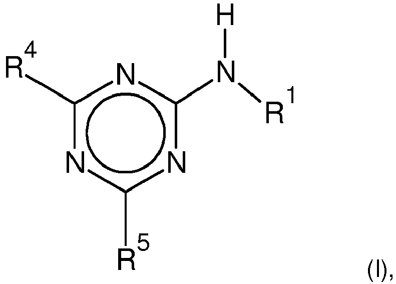 or
   at least one urea or urea derivative of the general formula (II) 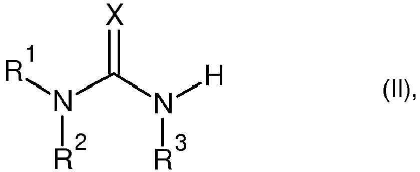 wherein
   - R⁴ and R⁵ mean independently from each other Q¹ or a moiety of the formula R⁶-N-R⁷ or R⁸-N-R⁹ bound with its central nitrogen atom to the triazine ring of the structure of formula (I), whereat
      - Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or -OC(O)O-,
   - R¹, R², R³, R⁶, R⁷, R⁸ and R⁹ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type -C(O)O-,-OC(O)-, -C(O)- and/or -OC(O) O- and/or can be functionalized by one or multiple hydroxyl groups, amino groups and/or mercapto groups, and
   - X means O or S,
is reacted
with at least one alcohol of the general formula (III)

R¹⁰-OH

wherein
R¹⁰ means a linear or branched C₁-C₂₀-alkyl, C₅-C₂₀-cycloalkyl, or C₁-C₂₀-alkylsubstituted C₅-C₂₀-aryl, which can be in each case interrupted by one or multiple oxygen atoms, sulphur atoms, substituted nitrogen atoms and/or by one or multiple groups of the type -C(O)O-, -OC(O)-, -C(O)- and/or -OC(O)O- and/or can be functionalized by one or multiple hydroxyl groups, amino groups and/or mercapto groups,
wherein
the reaction is carried out in the presence of at least one Ruthenate of the general formula (IV)

Mₙ(RuO₄)

and/or at least one Perruthenate of the general formula (V)

M(RuO₄)ₙ

wherein the cation M is selected from a group comprising an alkali, earth alkali or substituted or non-substituted ammonium cation and n= 1 or 2.

Thus, an alkylating method is provided that uses a ruthenate and/or a perruthenate compound as a catalyst. It is to be pointed out that the ruthenium is present in the catalyst as an anion (anion of the corresponding ruthenic acid). Ruthenate is the twice negatively charged oxyanion of ruthenium RuO₄²⁻; and perruthenate is the single negatively charged oxyanion of ruthenium RuO₄⁻ any salt or mineral containing this anion, formally derived from ruthenic acid.

The use of a ruthenate or perruthenate catalyst allows for the alkylation of a triazine, such as melamine, or urea with a high selectivity and low catalyst amounts. In case of melamine and methanol as starting materials a high conversion rate of over 90% and a high selectivity of over 90% for the N,N',N"-trimethylated melamine could be obtained. Thus, the use of ruthenate/perruthenate as catalyst leads to an almost complete suppression of side products and simultaneously to a high product yield. At the same time the amount of ruthenate/ perruthenate required is reduced in comparison in particular to the teaching of WO 2009/121603 A1 and WO 2009/121607 A1 which use catalysts containing a Ruthenium cation or Ruthenium in complex compounds, wherein Ruthenium has the oxidation state 0 (thus is present in metallic form).

In an embodiment of the present method the cation M is selected from a group comprising sodium Na⁺, potassium K⁺, or tetrapropylammonium N(C₃H₇)₄⁺.

Thus, the ruthenate may be selected from a group comprising K₂RuO₄. The perruthenate may be selected from a group comprising NaRuO₄, KRuO₄, and [N(C₃H₇)₄]RuO₄. The most preferred catalyst is potassium perruthenate KRuO₄.

It may also be possible that the applied ruthenate and/or perruthenate is polymer supported. This may be in particular the case when [N(C₃H₇)₄]RuO₄ is used.

However, it is preferred if the ruthenate and/or perruthenate catalyst does not require any carrier material. Thus, in the present method only a catalyst is preferably used that is free of any type of carrier material. Furthermore, preferably no binary catalytic system such as a mixture of two catalysts is used.

In a preferred embodiment the catalyst does not comprise any acidic carrier material, in particular no zeolithe, alumo silicate, alumo phosphate, metal oxide, silicate, layered silicate, aluminium oxide, silicon dioxide and/or carbon.

In a yet further preferred embodiment of the present method the amount of catalyst required is between 50 to 0.1 wt-%, preferably 30 to 0.5 wt-%, mostly preferably 20 to 1 wt-% in respect to the triazine/urea, in particular melamine, or urea. In a preferred embodiment 10wt% of the catalyst are used. This is in particular less catalyst required than in similar reactions described in the prior art, in particular WO 2009/121603 A1 und WO 2009/121607 A1.

In another variant of the present method the reaction is carried out in the presence of at least one gas selected from a group comprising ammonia, nitrogen, argon, helium, hydrogen, carbon dioxide and carbon monoxide. The use of hydrogen gas for providing the gas atmosphere of the reaction is in particular preferred. Thus, the present method may be carried out in a hydrogen gas atmosphere, preferably in a mixture of hydrogen gas and an inert gas such as nitrogen, argon or helium. Hydrogen is in particular preferred. This is due to the reaction mechanism that includes a dehydration step of the starting amine and a subsequent hydrogenation.

The reaction according to the present method is carried out in a pressure range between 1 bar and 100 bar, preferably between 5 bar and 50 bar. The reaction may be started under normal pressure or also at higher pressure of about 5 bar. It is to be understood that the pressure may increase in the course of the reaction in conjunction with a temperature increase.

In a further embodiment of the present method the reaction is carried out at a temperature between 100 °C and 300 °C, in particular 150 °C and 280 °C, in particular 180 °C and 250 °C.

In yet another preferred embodiment of the present method the reaction time is at least 2 hours, preferably at least 6 hours. Specifically the reaction time is between 2 and 12 hours, preferably between 5 and 10 hours, most preferably between 6 and 8 hours.

In a another variant of the present method the moieties R¹, R², R³, R⁶, R⁷, R⁸ and R⁹ of the triazine derivative of formula (I) or the urea compound of formula (II) are H, a linear or branched C₁-C₁₂-alkyl, a C₃-C₇-cycloalkyl and a linear or branched C₂-C₁₂-alkenyl.

It is even more preferred, if the moieties R¹, R², R³, R⁶, R⁷, R⁸ and R⁹ are H, a linear or branched C₁-C₆ alkyl, preferably a C₁, C₂, C₃ or C₄ alkyl.

In the most preferred variant the moieties R¹, R², R³, R⁶, R⁷, R⁸ and R⁹ are H. In this case melamine is used as preferred triazine.

Further examples for possible triazine derivatives as starting substances are benzoguanamine, acetoguanamine, 2,2-dimethylamino-4,6-diamino-1,3,5-triazine, 2,2-dibutylamino-4,6-diamino-1,3,5-triazine, 2,4,6-Tris-(2-hydroxyethyl)amino-1,3,5-triazine, 2-succinimido-4,6-diamino-1,3,5-triazine,. Examples for urea derivatives as starting substances are hydroxyethyl urea, N-methyl-urea and ethylene urea. Also non-derivatized urea can be used as starting substance.

In a variant at least one hydroxyl group of the reagent is bound to the alkyl moiety and not to the aryl moiety, if R¹⁰ means a C₁-C₂₀-alkylsubstituted C₅-C₂₀-aryl which can be interrupted or substituted as above. Thereby it can also be provided that all hydroxyl groups are bound to the alkyl moiety and not to the aryl moiety. In this manner the formation of arylated triazine or urea derivatives can be avoided in favour of the formation of arylsubstituted alkylated derivatives.

If hydroxyl groups of the alcohol are present on the alkyl moiety as well as on the aryl moiety in case R¹⁰ means a C₁-C₂₀-alkylsubstituted C₅-C₂₀-aryl, which can as previously be interrupted or substituted, the formation of arylsubstituted alkylated triazine and urea derivatives can still be preferred in respect to arylated derivatives by the selection of suitable reaction conditions. Thus, arylic hydroxyl groups react due to electronic influences of the aromatic general slower than hydroxyl groups bound to an alkyl moiety.

In another embodiment of the present method R¹⁰ is a linear or branched C₁-C₁₂-alkyl, preferably a linear or branched C₁-C₆₋-alkyl, most preferably C₁-C₄ alkyl such as methyl, ethyl, n-propyl, iso-propyl, n-butyl, iso-butyl.

It is furthermore preferred if the alcohol of formula (III) is a monoalcohol or a polyalcohol.

Examples for suitable monoalcohols are methanol, ethanol, propanol, isopropanol, butanol, hexanol, decanol, dodecanol, stearylalcohol, glycolmonomethylether, diethylenglycolmonomethylether and benzylalcohol.

Examples for suitable polyalcohohols are ethylenglycol, diethylenglycol, glycerol, trimethylolpropane, pentaerythrit, tripropylenglycol, trispropanolamine, triethanolamine, hexandiol, butandiol and glycerolmonostearat.

In a yet further embodiment a solvent may be used. The use of an appropriate solvent depends on the alcohol used as reaction partner. In case a low molecular weight alcohol that is liquid at room temperature such as methanol, ethanol or propanol is used as reaction partner no additional solvent may be added. In case a high molecular weight alcohol that is oily or solid at room temperature is used as reaction partner an additional solvent may be added.

In an embodiment of the method it is sufficient in case of short-chain alcohols as educts for the derivatization of the starting substance to apply the alcohol in excess in order to use said alcohol also as a solvent.

In a further variant inert solvents are used as solubilizer when using long-chain alcohols (more than 6, 8, 10 or 12 C-atoms) in order to achieve a better conversion. Basically, solubilizers can also be used with short-chain alcohols if those are for instance highly branched and have therefore a higher viscosity. The application of a solubilizer is always appropriate in such cases when the mixture cannot be stirred anymore without a solubilizer.

Examples for such solubilizers are tetrahydrofurane, diethylether, dimethoxymethane, dimethoxyethane, diethoxymethane, diethoxyethane, ethylenglycoldiethylether, ethylenglycoldibutylether, diethylenglycoldiethylether, dioxane, benzene, toluene, xylene, mesitylene, cumene, chlorbenzene, pentane, hexane, cyclohexane, heptane, octane, acetonitrile, methylacetate, ethylacetate, methylbenzoate, N,N-dimethylformamide, N,N-dimethylacetamide, N-methylpyrrolidone, 1,3-dimethylimidazolidinone.

It is recommended in case of a reagent with multiple hydroxyl groups to work strongly diluted in order to avoid side reactions by which oligomeric or polymeric compounds are formed from multiple triazine or urea units. For instance molecules can be formed from two triazine units in which the two triazine units are connected with each other via an ethylene bridge if a high concentration is used and glycol (1,2-ethandiol) is used as reagent. This is not desirable and can be avoided by reacting at diluted conditions.

In a preferred embodiment of the present method the compound obtained is a triazine derivative of the general formula (V) or a urea derivative of the general formula (VI): 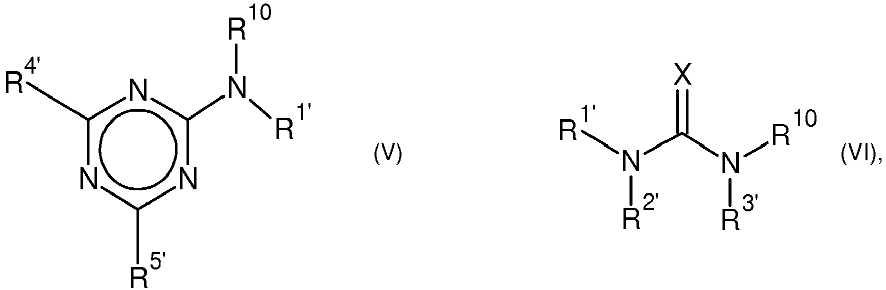 whereby
- R⁴ and R⁵ mean independently from each other Q¹ or a moiety of the formula R^{6'}-N-R^{7'}- or R^{8'}-N-R^{9'} being bound with its central nitrogen atom to the triazine ring of the structure of the formula (III), whereat
   - Q¹ means a linear or branched C₁-C₃₀-alkyl or a cyclic substituent in form of a C₅-C₂₀-cycloalkyl, a C₅-C₂₀-aryl, a C₁-C₂₀-alkyl substituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or -OC(O)O-,
- R^{1'}, R^{2'}, R^{3'}, R^{6'}, R^{7'}, and R⁹' mean independently from each other,
   - R¹⁰,
   - H
      or
   - linear or branched C₁-C₂₀-alkyl, C₅-C₂₀-cycloalkyl, C₅-C₂₀-aryl, C₁-C₂₀-alkyl substituted C₅-C₂₀-aryl, which in each case can be interrupted by one or multiple oxygen atoms, sulphur atoms and/or substituted nitrogen atoms and/or by one or multiple groups of the type -C(O)O-, -OC(O)-, -C(O)- and/or -OC(O)O-and/or can be functionalized by one or multiple hydroxy groups, amino groups and/or mercapto groups,
- R¹⁰ means a linear or branched C₁-C₂₀-alkyl, C₅-C₂₀-cycloalkyl or C₁-C₂₀-alkyl substituted C₅-C₂₀-aryl, which in each case can be interrupted by one or multiple oxygen atoms, sulphur atoms, substituted nitrogen atoms and/or by one or multiple groups of the type -C(O)O-, -OC(O)-, -C(O)- and/or -OC(O)O-, and/or can be functionalized by one or multiple hydroxyl groups, amino groups and/or mercapto groups, whereby R¹⁰ can form a cyclic structure with two covalent bonds bonded to the same nitrogen atom of the compounds of the general formula (III) or (IV), whereat one of the two covalent bonds is provided by one or two out of the moieties R^{1'}, R^{2'}, R^{3'}, R^{6'}, R^{7'}, R^{8'} or R^{9'},
- X means O or S.

A cyclic structure by the moiety R¹⁰ is formed for instance if an alcohol is used that carries at least two hydroxyl groups which react in each case with the same nitrogen atom of the starting compound.

In an embodiment of the method at least one moiety, in particular at least two moieties, in particular at least three moieties, in particular at least four moieties, in particular at least five moieties of the moieties R^{1'}, R^{2'}, R^{3'}, R^{6'}, R^{7'}, R^{8'} and R^{9'} have only the meaning of the moiety R¹⁰ and in particular not the meaning of H. This is in particular the case if the corresponding moieties R^{1'}, R^{2'}, R^{3'}, R^{6'}, R^{7'}, and R^{9'} have the meaning of a hydrogen moiety in the starting substance. In other words, several different amino groups of the formed compound (for instance one, two or three amino groups) can be substituted with the moiety R¹⁰ to a different degree (for instance once or twice) independently from each other.

In a most preferred embodiment at least three moieties R^{1'}, R^{2'}, R^{3'}, R^{6'}, R^{7'}, and R^{9'} have only the meaning of the moiety R¹⁰ and in particular not the meaning of H in case of triazine and at least two moieties R^{1'}, R^{2'}, R^{3'}, R^{6'}, R^{7'}, R^{8'} and R^{9'} have only the meaning of the moiety R¹⁰ and in particular not the meaning of H in case of urea. In other words, it is mostly preferred if each one of the three amino groups in case of a triazine (in particular melamine) and each one of the two amino groups in case of urea are substituted with one moiety R¹⁰.

Applying suitable experimental parameter compounds are obtained that comprise solely monosubstituted amino groups. For instance essentially pure N-monoalkyl compounds, N,N'-dialkyl compounds and N,N',N"-trialkyl compounds can be formed. Using suitable experimental parameters also essentially pure N-monohydroxyalkyl compounds, N,N'-dihydroxyalkyl compounds and N,N',N"-trihydroxyalkyl compounds, can be synthesized.

It is mostly preferred to obtain N-monosubstituted melamine, N,N'-disubstituted melamine, N,N', N"-trisubstituted melamine, N-monosubstituted urea and N, N'-disubstituted urea.

In an embodiment the formed compound is selected from the group comprising N-alkymelamin, N,N'-dialkylmelamine, and N,N',N"-trialkylmelamine, ,

In a further embodiment the formed compound is selected from the group comprising N-alkyl urea and N,N'-dialkylurea.

In a most preferred embodiment the present process allows for the formation of N,N',N"-trialkylmelamine or N,N'-dialkylurea to about 90%, preferably to about 95%, in particular preferably to 97%. The present process enables in a first step monoalkylation of a first amino group followed by monoalkylation of a second amino group and subsequently monoalkylation of a third amino group. Thus, the further alkylation or functionalisation of a secondary amino group is suppressed.

In a further embodiment the formed compound is selected from the group comprising N-alkylthiourea and N,N'-dialkylthiourea..

In an alternative embodiment the formed compound is selected from the group comprising N-alkylbenzoguanamine, and N,N'-dialkylbenzoguanamine .

In a further alternative embodiment the formed compound is selected from the group comprising N-alkylacetoguanamine, and N,N'-dialkylacetoguanamine .

In a further embodiment the formed compound is selected from the group comprising N-(hydroxyalkyl)-melamine, N,N'-di-(hydroxyalkyl)-melamine, and N,N',N"-tris-(hydroxyalkyl)-melamine.

Thereby in particular methyl, ethyl, butyl and/or hexyl moieties (or their mixtures) are used as alkyl moieties, but also all the other meanings of moiety R¹⁰ provided above. All moieties R¹⁰ carrying hydroxyl groups, for instance 2-hydroxyethyl-, hydroxypropyl- and/or hydroxyethoxyethyl moieties can be used as hydroxyalkyl moiety.

By the means of the following reaction equations an exemplary embodiment of the claimed method shall be explained in more detail. Thereby "T" means an increased temperature compared to the room temperature and "p" means an increased pressure in respect to the standard air pressure (specific parameter embodiments or reaction conditions and meanings of the moieties Rⁿ are explained further above):

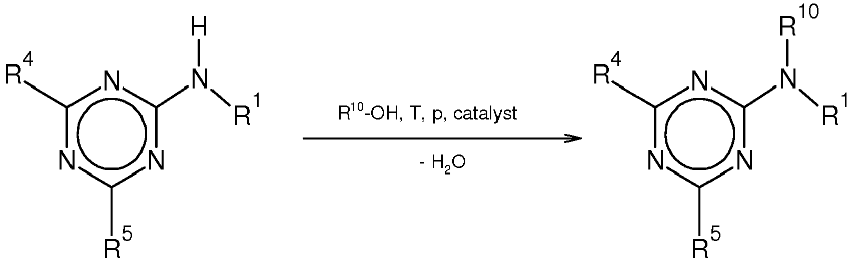

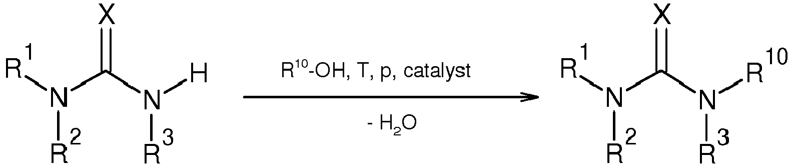

Due to the conversion of the starting substance a hydrogen atom bound to the nitrogen of an amino group is replaced by the moiety R¹⁰ of the used alcohol; a derivatization of the amino group occurs. After completion of the reaction the derivatized amino group has at least one substituent being different from a hydrogen atom, namely R¹⁰. Depending on the reaction conditions also further moieties Rⁿ, which previously had the meaning of a hydrogen atom, can be replaced by the moiety R¹⁰. In this manner compounds with different degrees of substituted amino groups can be obtained.

Due to the obtained purity the derivatized, in particular alkylated, compounds obtained according to this method as for instance alkylated amino triazines and alkylated ureas can be used as formaldehyde resins. Under the meaning "formaldehyde resin" a resin made of aldehyde like formaldehyde and the corresponding formed compound is to be understood. These aldehyde or specifically formaldehyde resins have specific properties in respect to rheology, hydrophilicity or lipophilicity and surface properties. They are in particular suitable for application in the area of the laminate coating of the wood processing industry.

In particular alkylated compounds as for instance the symmetrical trialkyl melamine (N,N',N"-trialkyl melamine) are also suitable as cross linker. Further areas of use of the formed alkylated aminotriazines and the alkylated ureas are the area of additives for plasticization, the area of flame retardant additives, the area of comonomers for a polyurethane and the area of agrochemicals.

Since due to an alkylation (in particular if longer-chained alkyl moieties are bound to the starting substance having an amino group) the hydrophobicity of the formed compound is increased compared to the starting substance, the compounds obtained by the claimed method can be used in a mixture with a polyolefine, in particular a polyethylene (polyethen) or polypropylene (polypropen). In this manner the flame retardant properties or surface properties of an object made of a mixture of the polyolefin and the formed compound can be for instance improved compared to the corresponding properties of an object made if an unmodified polyolefin.

Further details of the invention are explained in more detail by the means of the following examples.

### Example 1:

2.5 g melamine, 105 g methanol and 0.25 g potassium perruthenate were mixed in a beaker and filled into the autoclave. The autoclave was closed and the stirrer was turned on with 500 rpm. After flushing with helium, hydrogen was flushed through the autoclaved and then it was closed with atmospheric pressure of hydrogen inside. The reaction mixture was heated up to 250 °C and held at this temperature for 6 hours. After cooling down to room temperature and flushing with helium the autoclave was opened and the reaction mixture was transferred into a beaker. The catalyst was separated with a centrifuge of and the solvent methanol was evaporated in a Rotavapor. N,N',N"-Trimethylmelamine is the main product (see reaction scheme below). The conversion is 97 % with a selectivity of 90 %.

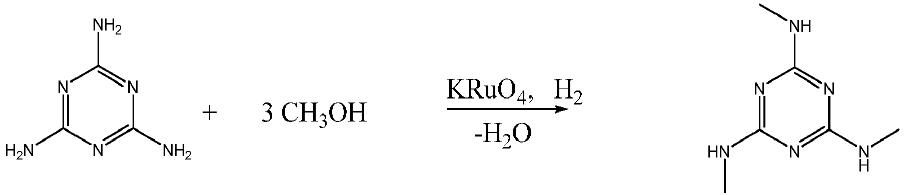

### Example 2:

2.5 g melamine, 105 g ethanol and 0.25 g potassium perruthenate were mixed in a beaker and filled into the autoclave. The autoclave was closed and the stirrer was turned on with 500 rpm. After flushing with helium, hydrogen was flushed through the autoclaved and then it was closed with atmospheric pressure of hydrogen inside. The reaction mixture was heated up to 250 °C and held at this temperature for 6 hours. After cooling down to room temperature and flushing with helium the autoclave was opened and the reaction mixture was transferred into a beaker. The catalyst was separated with a centrifuge of and the solvent methanol was evaporated in a Rotavapor. N,N',N"-Triethylmelamine is the main product (see reaction scheme below). The conversion is 95 % with a selectivity of 87 %.

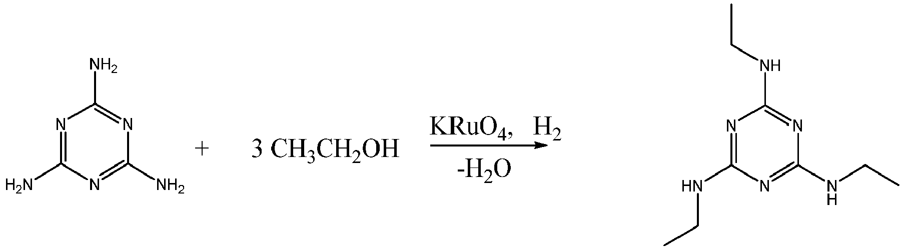

### Example 3:

2.5 g melamine, 105 g propanol and 0.25 g potassium perruthenate were mixed in a beaker and filled into the autoclave. The autoclave was closed and the stirrer was turned on with 500 rpm. After flushing with helium, hydrogen was flushed through the autoclaved and then it was closed with atmospheric pressure of hydrogen inside. The reaction mixture was heated up to 250 °C and held at this temperature for 6 hours. After cooling down to room temperature and flushing with helium the autoclave was opened and the reaction mixture was transferred into a beaker. The catalyst was separated with a centrifuge of and the solvent methanol was evaporated in a Rotavapor. N,N',N"-Tripropylmelamine is the main product (see reaction scheme below). The conversion is 93 % with a selectivity of 86 %.

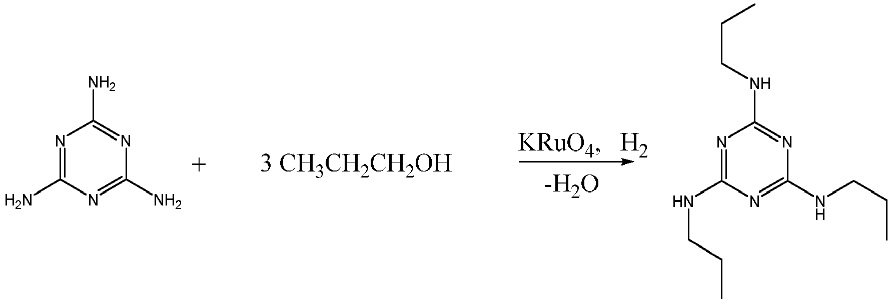

### Example 4:

2.5 g urea, 105 g propanol and 0.25 g potassium perruthenate were mixed in a beaker and filled into the autoclave. The autoclave was closed and the stirrer was turned on with 500 rpm. After flushing with helium, hydrogen was flushed through the autoclaved and then it was closed with atmospheric pressure of hydrogen inside. The reaction mixture was heated up to 125 °C and held at this temperature for 6 hours. After cooling down to room temperature and flushing with helium the autoclave was opened and the reaction mixture was transferred into a beaker. The catalyst was separated with a centrifuge of and the solvent methanol was evaporated in a Rotavapor. N,N'-dimethylurea is the main product (see reaction scheme below). The conversion is 89 % with a selectivity of 87 %.

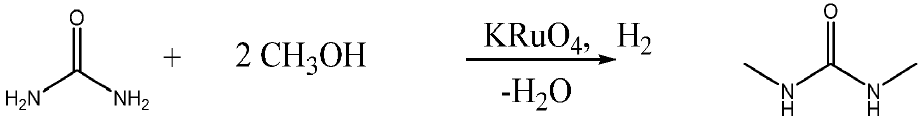

## Claims

1. Method for alkylating an amino compound, **characterized in that**
- at least one triazine derivative of general formula (I) 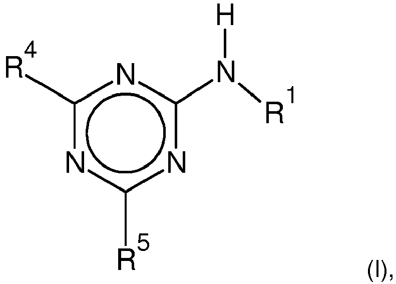 or
at least one urea or urea derivative of the general formula (II) 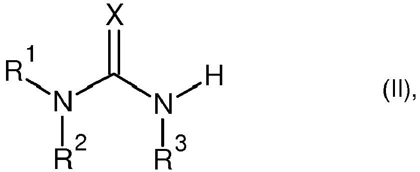 wherein
• R⁴ and R⁵ mean independently from each other Q¹ or a moiety of the formula R⁶-N-R⁷ or R⁸-N-R⁹ bound with its central nitrogen atom to the triazine ring of the structure of formula (I), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or -OC(O)O-,
• R¹, R², R³, R⁶, R⁷, R⁸ and R⁹ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type -C(O)O-,-OC(O)-, -C(O)- and/or -OC(O) O- and/or can be functionalized by one or multiple hydroxyl groups, amino groups and/or mercapto groups, and
• X means O or S,
is reacted
with at least one alcohol of the general formula (III)
R¹⁰-OH
wherein
R¹⁰ means a linear or branched C₁-C₂₀-alkyl, C₅-C₂₀-cycloalkyl, or C₁-C₂₀-alkylsubstituted C₅-C₂₀-aryl, which can be in each case interrupted by one or multiple oxygen atoms, sulphur atoms, substituted nitrogen atoms and/or by one or multiple groups of the type -C(O)O-, -OC(O)-, -C(O)- and/or -OC(O)O- and/or can be functionalized by one or multiple hydroxyl groups, amino groups and/or mercapto groups,
wherein
the reaction is carried out in the presence of at least one Ruthenate of the general formula (IV)
Mₙ(RuO₄)
and/or at least one Perruthenate of the general formula (V)
M(RuO₄)ₙ
wherein the cation M is selected from a group comprising an alkali, earth alkali or substituted or non-substituted ammonium cation.

2. Method according to claim 1, **characterized in that** the cation M is selected from a group comprising sodium Na⁺, potassium K⁺, or tetrapropylammonium N(C₃H₇)₄⁺.

3. Method according to claim 1 or 2, **characterized in that** the perruthenate is potassium perruthenate KRu04.

4. Method according to one of the preceding claims, **characterized in that** the amount of catalyst required is between 50 and 0.1 wt%, preferably 30 and 0.5 wt%, mostly preferably 20 and 1 wt% in respect to the triazine, in particular melamine, or urea.

5. Method according to one of the preceding claims, **characterized in that** the reaction is carried out in the presence of at least one gas selected from a group comprising ammonia, nitrogen, argon, helium, hydrogen, carbon dioxide and carbon monoxide.

6. Method according to one of the preceding claims, **characterized in that** the reaction is carried out in a pressure range between 1 bar and 100 bar, preferably between 5 and 50 bar.

7. Method according to one of the preceding claims, **characterized in that**, the reaction is carried out at a temperature between 100°C and 300°C, in particular 150°C to 280°C, in particular 180 °C to 250 °C.

8. Method according to one of the preceding claims, **characterized in that** the reaction time is at least 2 hours, preferably at least 6 hours.

9. Method according to one of the preceding claims, **characterized in that** R¹, R², R³, R⁶, R⁷, and R⁹ are H, a linear or branched C₁-C₁₂-alkyl, a C₃-C₇-cycloalkyl and a linear or branched C₂-C₁₂-alkenyl.

10. Method according to one of the preceding claims **characterized in that** R¹, R², R³, R⁶, R⁷, and R⁹ are H, a linear or branched C₁-C₆ alkyl, preferably a C₁, C₂, C₃ or C₄ alkyl.

11. Method according to one of the preceding claims, **characterized in that** R¹, R², R³, R⁶, R⁷, and R⁹ are H.

12. Method according to one of the preceding claims, **characterized in that** at least one hydroxyl group of the alcohol is present on the alkyl moiety and not on the aryl moiety if R¹⁰ means a C₁-C₂₀-alkyl substituted C₅-C₂₀-aryl.

13. Method according to one of the preceding claims, **characterized in that** the alcohol is a monoalcohol or a polyalcohol.

14. Method according to one of the preceding claims, **characterized in that** the produced compound is a triazine derivative of the general formula (V) or a urea derivative of the general formula (VI): 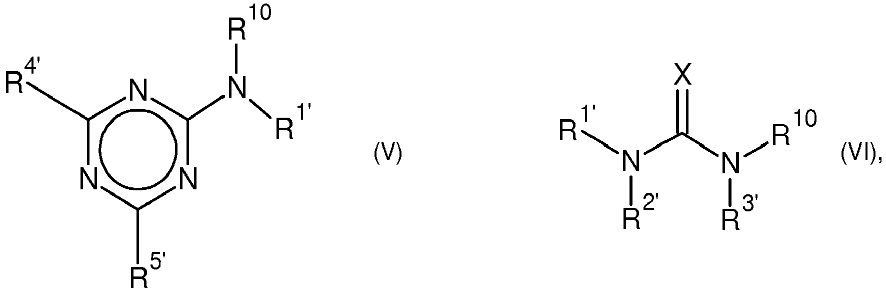 whereby
• R^{4'} and R^{5'} mean independently from each other Q¹ or a moiety of the formula R^{6'}-N-R^{7'}- or R^{8'}-N-R^{9'} being bound with its central nitrogen atom to the triazine ring of the structure of the formula (III), whereat
- Q¹ means a linear or branched C₁-C₃₀-alkyl or a cyclic substituent in form of a C₅-C₂₀-cycloalkyl, a C₅-C₂₀-aryl, a C₁-C₂₀-alkyl substituted C₅-C₂₀-aryl or an amide of a cyclic unsaturated carboxylic acid, whereat the C₁-C₃₀-alkyl or the cyclic substituent can be interrupted by one or multiple oxygen atoms, sulphur atoms, substituted nitrogen atoms and/or by one or multiple groups of the type -C(O)O-,-OC(O)-,-C(O)- and/or -OC(O)O-,
• R^{1'}, R^{2'}, R^{3'}, R^{6'}, R^{7'}, and R^{9'} mean independently from each other,
- R¹⁰,
- H
, or
- linear or branched C₁-C₂₀-alkyl, C₅-C₂₀-cycloalkyl, C₅-C₂₀-aryl, C₁-C₂₀-alkyl substituted C₅-C₂₀-aryl, which in each case can be interrupted by one or multiple oxygen atoms, sulphur atoms and/or substituted nitrogen atoms and/or by one or multiple groups of the type -C(O)O-, -OC(O)-, -C(O)- and/or -OC(O)O- and/or can be functionalized by one or multiple hydroxy groups, amino groups and/or mercapto groups,
• R¹⁰ means a linear or branched C₁-C₂₀-alkyl, C₅-C₂₀-cycloalkyl or C₁-C₂₀-alkyl substituted C₅-C₂₀-aryl, which in each case can be interrupted by one or multiple oxygen atoms, sulphur atoms, substituted nitrogen atoms and/or by one or multiple groups of the type -C(O)O-, -OC(O)-, -C(O)- and/or -OC(O)O-, and/or can be functionalized by one or multiple hydroxyl groups, amino groups and/or mercapto groups, whereby R¹⁰ can form a cyclic structure with two covalent bonds bonded to the same nitrogen atom of the compounds of the general formula (III) or (IV), whereat one of the two covalent bonds is provided by one of the moieties R^{1'}, R^{2'}, R^{3'}, R^{6'}, R^{7'}, R^{8'} or R^{9'},
• X means O or S.

15. Method according to one of the preceding claims, **characterized in that** the produced compounds are N-monosubstituted melamine, N,N'-disubstituted melamine, N,N',N"-trisubstituted melamine, N-monosubstituted urea and N, N'-disubstituted urea.
